# EUROPÄISCHE PATENTANMELDUNG

(11) **EP 0 811 424 A2**
(43) Veröffentlichungstag der Anmeldung: **10.12.1997**
(21) Anmeldenummer: 97107515.5
(22) Anmeldetag: 07.05.1997
(51) Int. Cl.: B01J 31/24, C07C 45/50

(54) **Katalysatorsysteme auf der Basis von Rhodium-Komplex-verbindungen mit Diphosphin-Liganden und ihre Verwendung bei der Herstellung von Aldehyden**

(30) Priorität: 15.05.1996 DE 19619527
(71) Anmelder: HOECHST AKTIENGESELLSCHAFT, 65926 Frankfurt am Main (DE)
(72) Erfinder: Bahrmann, Helmut, Dr., 46499 Hamminkeln (DE); Lappe, Peter, Dr., Plano, TX 75025 (US); Müller, Thomas, 46535 Dinslaken (DE)

(57) **Zusammenfassung**

Die Erfindung betrifft neue Katalysatorsysteme auf der Basis von wasserunlöslichen Rhodium-Komplexverbindungen mit Diphosphin-Liganden. Die Diphosphin-Liganden liegen als Ammoniumcarboxylate, -sulfonate oder -phosphonate mit ein- bzw. mehrfach geladenem Diphosphin-Anion und der korrespondierenden Anzahl Ammonium-Kationen als Gegenionen vor.

Die Erfindung betrifft ferner die Verwendung der neuen Katalysatorsysteme bei der Herstellung von Aldehyden durch Umsetzung von olefinisch ungesättigten Verbindungen mit Wasserstoff und Kohlenmonoxid in homogener Phase.

## Beschreibung

Die Erfindung betrifft neue Katalysatorsysteme auf der Basis von Rhodium-Komplexverbindungen mit Diphosphin-Liganden und ihre Verwendung bei der Herstellung von Aldehyden durch Umsetzung von olefinisch ungesättigten Verbindungen mit Wasserstoff und Kohlenmonoxid in homogener Phase.

Phosphine werden in den verschiedensten industriell ausgeübten Verfahren eingesetzt. Von Bedeutung ist besonders ihr Einsatz als Liganden für Metallkomplexkatalysatoren, die als Zentralatom bevorzugt ein Metall der VIII. Gruppe des Periodensystems der Elemente sowie gegebenenfalls neben den Phosphin-Liganden noch weitere, zur Komplexbildung fähige Gruppen enthalten.

Die technisch in großem Umfang durchgeführte Hydroformylierung von Olefinen erfolgt zunehmend in Gegenwart von Katalysatorsystemen auf der Basis von Rhodium-Komplexverbindungen, die tertiäre Phosphine oder Phosphite als Liganden enthalten. Da diese Liganden in der Regel im Überschuß vorliegen, besteht das Katalysatorsystem aus Komplexverbindung und zusätzlichem reinem Ligand. Entsprechend der Löslichkeit dieser Katalysatorsysteme in organischen Medien erfolgt die Hydroformylierung in homogener Phase. Zur Abtrennung der Reaktionsprodukte und Wiedergewinnung der im Reaktionsprodukt homogen gelösten Katalysatoren destilliert man das Reaktionsprodukt im allgemeinen aus dem Reaktionsgemisch ab. Dies ist aber wegen der thermischen Empfindlichkeit der gebildeten Aldehyde nur bei der Hydroformylierung von niederen Olefinen mit bis zu etwa 8 Kohlenstoffatomen im Molekül möglich. Bei der Hydroformylierung langkettiger Olefine oder olefinischer Verbindungen mit funktionellen Gruppen werden Produkte mit hohem Siedepunkt gebildet, die sich destillativ nicht mehr zufriedenstellend vom Katalysator abtrennen lassen: Die thermische Belastung des Destillationsgutes führt durch Dickölbildung zu erheblichen Verlusten an Wertprodukt sowie durch Zersetzung der Komplexverbindungen zu Verlusten an Katalysator. Hierdurch wird die wirtschaftliche Attraktivität des Verfahrens entscheidend verringert.

Aus EP-A-0 374 615 ist es bekannt, daß sich metallorganische Komplexverbindungen, die Phosphor(III)verbindungen als Liganden enthalten, aus organischen Lösungsmitteln unter Einsatz selektiver semipermeabler Polyaramid-Trennmembranen unversehrt, d.h. ohne Abbau der katalytisch aktiven Metallverbindung, abtrennen und zurückgewinnen lassen. Als treibende Kraft für den Trennvorgang kann hierbei sowohl eine Druckdifferenz (Druckfiltration) als auch eine Konzentrationsdifferenz (Dialyse) dienen. Das Verfahren eignet sich besonders zur Abtrennung von metallorganischen Komplexverbindungen und/oder Metallcarbonylen mit Phosphor(III)verbindungen als Liganden aus organischen Lösungen, in denen sie zuvor Anwendung als homogene Katalysatoren gefunden haben. Als Rhodiumkomplexverbindungen sind in EP-A-0 374 615 HRhCO[P(C₆H₅)₃]₃, RhCl[P(C₆H₅)₃]₃ und solche Verbindungen genannt, die als Liganden Alkyl- oder Arylammoniumsalze sulfonierter oder carboxylierter Triarylphosphine der allgemeinen Formel 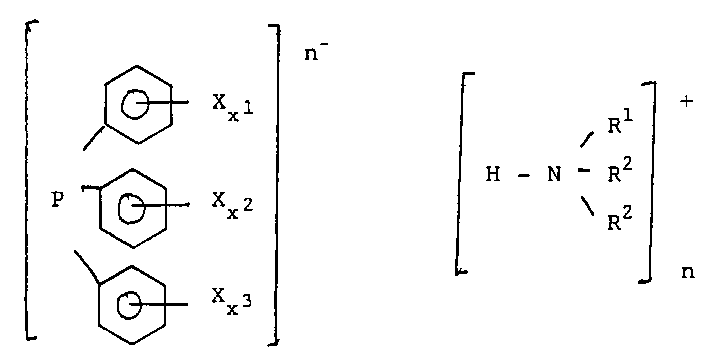 enthalten, wobei X einen Sulfonatrest(SO₃⁻) oder Carboxylatrest(COO⁻) bedeutet, x¹, x² und x³ 0 oder 1 sind, R¹ und R² jeweils gleiche oder verschiedene C₄ - C₁₂-Alkylreste, C₆ - C₁₂-Arylreste oder C₆ - C₁₂-Cycloalkylreste bedeuten und R¹ zusätzlich auch für Wasserstoff stehen kann.

Derartige Rhodiumkomplexverbindungen mit Alkyl- oder Arylammoniumsalzen sulfonierter oder carboxylierter Triarylphosphine als Liganden werden zur Hydroformylierung olefinisch ungesättigter Verbindungen in homogener Phase eingesetzt, benötigen aber zu ihrer Stabilisierung einen großen Überschuß an freien, nicht komplexierten Liganden.

Dieser große Ligandenüberschuß führt im Hydroformylierungsgemisch wiederum zu einer hohen Salzkonzentration, die die Umsetzung des Olefins mit Kohlenmonoxid und Wasserstoff ungünstig beeinflussen kann, da sie die Löslichkeit der Reaktionspartner im Reaktionsgemisch beeinträchtigt und zudem eine Schaumbildung fördert. Auch bei einer Membranfiltration, die nach der Hydroformylierung zur Abtrennung des Katalysatorsystems vom Reaktionsprodukt durchgeführt werden kann, wirkt sich der hohe Ligandenüberschuß, d.h. die hohe Salzkonzentration negativ aus; zum einen verringert sie die Flußleistung der Membranfiltration, was nur durch die Verwendung von sehr viel größeren Membranflächen wieder ausgeglichen werden kann, zum anderen verringert sie den maximalen Grad der Aufkonzentration des Retentats. Dies führt bei einer Rezirkulierung des katalysatorhaltigen Retentats in die Hydroformylierungsreaktion zu einer Reduzierung des für die übrigen Reaktanden zur Verfügung stehenden Reaktorvolumens, ein Effekt, der ebenso wie die großen Membranflächen erhöhte Verfahrenskosten bedingt und damit die wirtschaftliche Attraktivität des Verfahrens mindert.

Auch im Falle einer destillativen Aufarbeitung des Hydroformylierungsgemisches sind die hohen Salzkonzentrationen störend, da sie zu einem erhöhten Anteil an salzhaltigem Dicköl führen.

Es bestand daher die Aufgabe, neue Katalysatorsysteme auf der Basis von Rhodium-Komplexverbindungen mit phosphororganischen Liganden zur Verfügung zu stellen, die bei der Hydroformylierung von olefinisch- ungesättigten Verbindungen in homogener Phase eingesetzt werden können, hierbei zu hohen Aktivitäts- und Selektivitätswerten führen und auf einfache Weise aus dem Reaktionsgemisch der Hydroformylierung abgetrennt werden können.

Gelöst wird diese Aufgabe durch Katalysatorsysteme auf der Basis von Rhodium-Komplexverbindungen mit Diphosphinen als Liganden, dadurch gekennzeichnet, daß als Diphosphine Verbindungen der allgemeinen Formel I verwendet werden 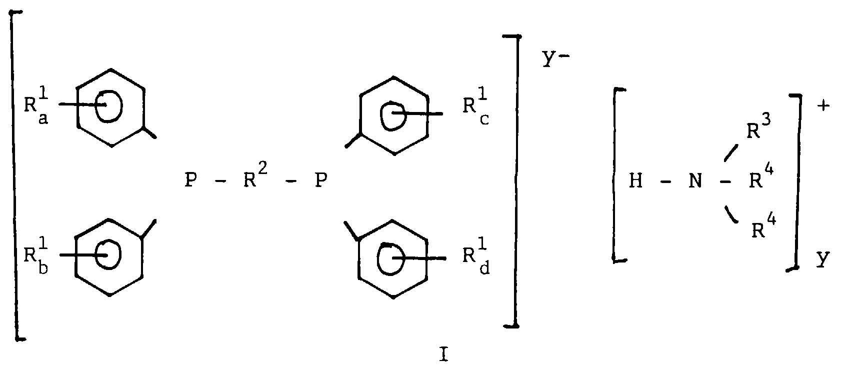 worin R¹ einen Carboxylat-(COO⁻), Sulfonat-(SO₃⁻), Phosphonat-(PO₃²⁻) oder 2-Aminoethanbisphosphonat-Rest ⁅NH-CH₂-CH(PO₃²⁻)2] darstellt, R² für einen geradkettigen Alkylenrest mit 1 - 8 Kohlenstoffatomen, einen Sauerstoff enthaltenden Alkylenrest mit 2 - 6 Kohlenstoffatomen, einen Cycloalkylenrest mit 3 bis 10 Kohlenstoffatomen oder einen Rest der Formeln II, III, IV oder V 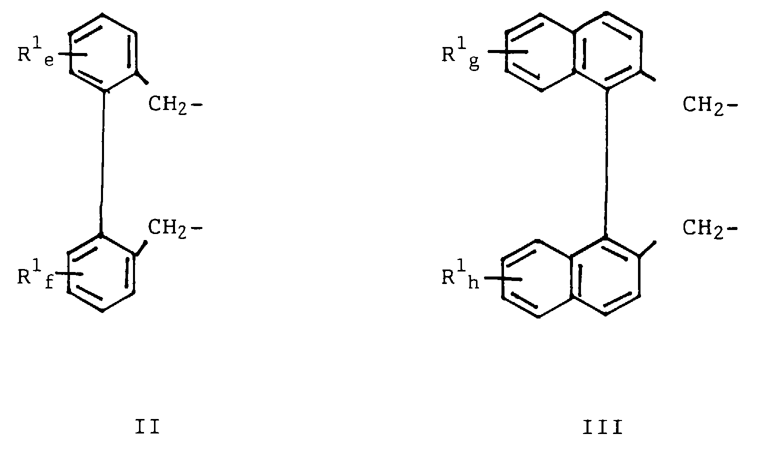 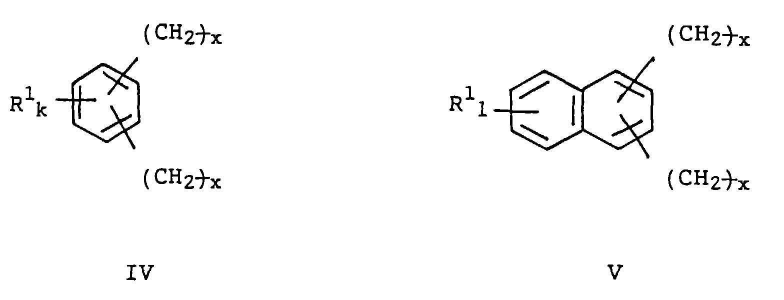 steht, a, b, c, d, e, f, g, h, k und l gleich oder verschieden und 0 oder 1 sind, wobei mindestens einer der Parameter a, b, c, d, e, f, g, h, k oder l gleich 1 sein muß, x gleich oder verschieden und 0 oder 1 ist, R³ und R⁴ gleich oder verschieden sind und für C₄ - C₂₆-Alkyl-, substituierte oder unsubstituierte C₆ - C₁₀-Aryl- oder C₆ - C₁₀-Cycloalkylreste oder einen Benzylrest stehen und R³ auch Wasserstoff bedeuten kann.

Die Verbindungen der allgemeinen Formel I liegen als Ammoniumcarboxylate, -sulfonate oder -phosphonate mit ein- bzw. mehrfach geladenem Diphosphin-Anion und der korrespondierenden Anzahl Ammonium-Kationen als Gegenionen vor. Sie sind in Wasser im allgemeinen nicht oder nur in sehr geringem Maß löslich. In organischen Lösungsmitteln besitzen sie dagegen eine gute bis sehr gute Löslichkeit und eignen sich daher besonders für den Einsatz in organischer Phase.

Aufgrund des Einbaus zweier trivalenter Phosphor-Atome lassen sich die Verbindungen der allgemeinen Formel I hervorragend als chelatbildende Liganden in den erfindungsgemäßen Katalysatorsystemen verwenden.

In der allgemeinen Formel I bedeutet R¹ einen Carboxylat-, Sulfonat-, Phosphonat- oder 2-Aminoethanbisphosphonatrest, bevorzugt einen Sulfonatrest.

R² steht für einen geradkettigen Alkylenrest mit 1 - 8, bevorzugt 1 - 5 und insbesondere 1 - 3 Kohlenstoffatomen. R² kann ferner einen Sauerstoff enthaltenden Alkylenrest mit 2 - 6, bevorzugt mit 2 - 4 Kohlenstoffatomen und insbesondere mit 4 Kohlenstoffatomen gemäß der Formel (̵CH₂)̵₂O(̵CH₂)̵₂ darstellen. R² steht ebenfalls für einen Cycloalkylenrest mit 3 - 10, bevorzugt 6 - 10 Kohlenstoffatomen oder für einen Rest der Formeln II, III, IV oder V, bevorzugt der Formel II.

In den allgemeinen Formeln I, II, III, IV und V sind a, b, c, d, e, f, g, h, k und l gleich oder verschieden und 0 oder 1, wobei mindestens einer der Parameter a, b, c, d, e, f, g, h, k oder l gleich 1 sein muß. In Verbindungen der Formel I, in denen R² einen Rest der Formel II darstellt, ist die Summe aus a, b, c, d, e und f, welche die Zahl der Reste R¹ angibt, bevorzugt 1 - 3; ist R² ein Rest der Formel III, so stellt die Summe aus a, b, c, d, g und h bevorzugt eine Zahl von 1 - 2 dar. Ist R² ein Rest der Formel IV oder V, so ist die Summe aus a, b, c, d und k bzw. a, b, c, d und l bevorzugt 1 - 3. Hat R² die Bedeutung eines geradkettigen Alkylenrestes mit 1 - 8 Kohlenstoffatomen, eines Sauerstoff enthaltenden Alkylenrestes mit 2 - 6 Kohlenstoffatomen oder eines Cycloalkylenrestes mit 3 - 10 Kohlenstoffatomen, so ist die Summe aus a, b, c und d bevorzugt eine Zahl von 2 - 4.

In der allgemeinen Formel I sind R³ und R⁴ gleich oder verschieden und stehen für C₄ - C₂₆-, bevorzugt C₁₈ - C₂₂-Alkyl-, substituierte oder unsubstituierte C₆ - C₁₀-Aryl-, bevorzugt Phenyl-, C₆ - C₁₀-Cycloalkyl-, bevorzugt Cyclohexylreste oder einen Benzylrest. R³ kann auch Wasserstoff bedeuten. Somit leiten sich die Ammonium-Kationen [H-NR³R⁴R⁴]⁺ von sekundären oder tertiären Aminen NR³R⁴R⁴ ab, die insgesamt 8 - 78, bevorzugt 12 - 72, besonders bevorzugt 21 bis 60 und insbesondere 36 bis 54 Kohlenstoffatome in den Substituenten R³ und R⁴ enthalten. Bevorzugt leiten sich die Ammonium-Kationen von Di-2-ethylhexylamin, Tri-n-octylamin, Triisooctylamin, Triisononylamin, Triisodecylamin, Distearylamin, Methyldistearylamin, Tricetylamin oder Trieicosylamin ab.

Als besonders geeignete Diphosphin-Anionen in der allgemeinen Formel I seien sulfonierte Diphosphin-Anionen wie Bis(disulfonatophenylphosphino)methan
1,2-Bis(disulfonatophenylphosphino)ethan
1,3-Bis(disulfonatophenylphosphino)propan
1,4-Bis(disulfonatophenylphosphino)butan
1,5-Bis(disulfonatophenylphosphino)pentan
Bis-(diphenylphosphinomethyl)ether
Bis-(diphenylphosphinoethyl)ether
genannt.

Die Verbindungen der allgemeinen Formel I, wobei R¹ ein Sulfonatrest ist, können hergestellt werden, indem man das sekundäre Phosphinoxid der Formel VI 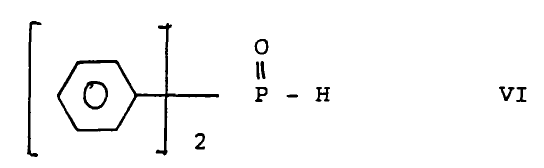 in Gegenwart einer Base mit einem Dihalogenid der Formel VII

X - R² - X VII

worin R² die in zuvor genannten Bedeutungen hat und X für einen Halogenrest, bevorzugt Chlor oder Brom steht, gegebenenfalls in Anwesenheit eines Lösungsmittels bei -20 bis 100°C zu einem Diphosphinoxid der Formel VIII 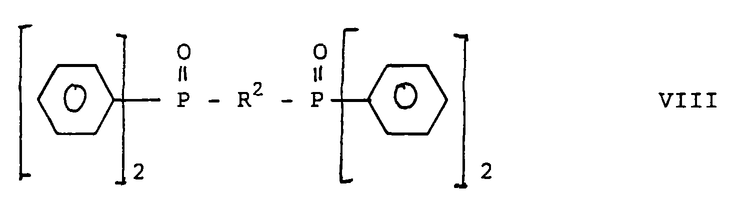 umsetzt, dieses Diphosphinoxid der Formel VIII gegebenenfalls in Gegenwart eines Lösungsmittels mit einem Silan der Formel HSiClₘR⁵ₙ, wobei m gleich 2 oder 3 ist, n gleich 0 oder 1 ist, m + n gleich 3 ist und R⁵ für einen Methyl- oder Phenylrest steht, bei 80 - 160°C zu einem Diphosphin der Formel IX 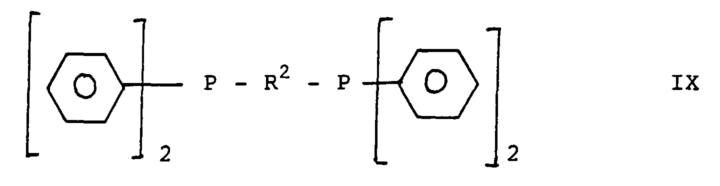 reduziert und dieses Diphosphin der Formel IX bei 0 bis 50°C mit Oleum sulfoniert, das Sulfonierungsgemisch mit Wasser verdünnt und das in einem wasserunlöslichen organischen Lösungsmittel gelöste wasserunlösliche Amin NR³R⁴R⁴, von dem sich das Ammonium-Kation [H-NR³R⁴R⁴]⁺ in Formel I ableitet, zugibt.

Dieses Syntheseverfahren ist ausführlich in einer am gleichen Tag wie die vorliegende Anmeldung eingereichten deutschen Patentanmeldung beschrieben.

Gegenstand der Erfindung ist ferner ein Verfahren zur Herstellung von Aldehyden durch Umsetzung von olefinisch ungesättigten Verbindungen mit Kohlenmonoxid und Wasserstoff in Gegenwart des vorstehend beschriebenen Katalysatorsystems auf der Basis von Rhodium-Komplexverbindungen mit Diphosphin-Liganden der allgemeinen Formel I.

Die Bildung des erfindungsgemäßen Katalysatorsystems aus Rhodium oder einer Rhodium-Verbindung und der Diphosphin-Verbindung der Formel I erfolgt entweder in einem der Hydroformylierung vorgeschalteten Schritt, der sogenannten Präformierung, oder aber, insbesondere bei kontinuierlicher Arbeitsweise, in-situ während der Hydroformylierungsreaktion.

Die der Hydroformylierung vorgeschaltete Präformierung erfolgt bevorzugt bereits in dem Reaktor, in dem nachfolgend auch die Hydroformylierung stattfindet, sie kann jedoch auch in einem separaten Reaktionsbehälter durchgeführt werden.

Zur Herstellung des Katalysatorsystems durch Präformierung wird die Rhodium-Komponente (Rhodium oder eine Rhodiumverbindung) mit der Diphosphin-Verbindung gemäß Formel I entweder im Hydroformylierungsreaktor oder in der separaten Vorrichtung zusammengebracht. Hierbei setzt man sowohl das Rhodium oder die Rhodiumverbindung als auch die Diphosphin-Verbindung gemäß Formel I in einem organischen Lösungsmittel gelöst oder, im Falle von elementarem Rhodium, suspendiert ein. Geeignete Lösungsmittel sind hierbei organische Lösungsmittel, die unter den Bedingungen der nachfolgenden Hydroformylierung inert sind, wie Toluol, ortho-Xylol, meta-Xylol, para-Xylol, Gemische isomerer Xylole, 2-Ethylhexanol, Ethylbenzol, Mesitylen, Gemische dieser Verbindungen oder aliphatische Kohlenwasserstoffe. Bevorzugt wird 2-Ethylhexanol eingesetzt.

Das Rhodium/Diphosphin-Gemisch wird anschließend mit einem Gemisch aus Kohlenmonoxid und Wasserstoff beaufschlagt und unter einem Kohlenmonoxid/Wasserstoff-Druck von 15 - 25 MPa bei Temperaturen von 80 - 150°C mindestens 1 h umgesetzt unter Bildung von wasserunlöslichen und in organischen Medien löslichen Rhodium-Komplexverbindungen, die das Diphosphin als Liganden enthalten. Sie ergeben zusammen mit dem im organischen Lösungsmittel gelösten Diphosphin-Überschuß das Katalysatorsystem. Die Lösung des Katalysatorsystems kann dann, sofern die Herstellung in einer separaten Vorrichtung erfolgt, in den Hydroformylierungsreaktor überführt werden und mit dem zu hydroformylierenden Olefin versetzt werden.

Soll die Herstellung des Katalysatorsystems in-situ während der Hydroformylierungsreaktion erfolgen, so werden die oben beschriebenen Komponenten, Rhodium bzw. Rhodiumverbindung und Diphosphin, gleichzeitig mit dem Olefin in den Hydroformylierungsreaktor eingebracht.

Es hat sich bei der Bildung des Katalysatorsystems bewährt, Rhodium und die Diphosphine der Formel I nicht in stöchiometrischem Verhältnis, also entsprechend der chemischen Zusammensetzung der sich bildenden Rhodium-Komplexverbindung zu verwenden, sondern die Diphosphine im Überschuß einzusetzen. Dabei kann man das Verhältnis von Rhodium und Diphosphin in weiten Grenzen variieren und je mol Rhodium etwa 1 bis 100 mol Diphosphin anwenden. Bevorzugt wird ein Verhältnis von Rhodium zu Diphosphin von 1 : (1 - 45). Besonders bevorzugt ist ein Verhältnis von 1 : (1 - 25), und insbesondere wird ein Verhältnis von 1 : (1 - 10) verwendet.

Rhodium gelangt entweder als Metall oder als Verbindung zum Einsatz. In metallischer Form verwendet man es in Form feinverteilter Partikel oder in dünner Schicht auf einem Träger wie Aktivkohle, Calciumcarbonat, Aluminiumsilikat oder Tonerde niedergeschlagen. Als Rhodiumverbindungen kommen solche Substanzen in Betracht, die in organischen Lösungsmitteln löslich oder suspendierbar sind oder unter den Reaktionsbedingungen darin löslich oder suspendierbar werden. Geeignet sind die verschiedenen Rhodiumoxide, Salze anorganischer Wasserstoff- oder Sauerstoffsäuren, sowie Salze aliphatischer Mono- oder Polycarbonsäuren. Beispiele für Rhodiumsalze sind Rhodiumnitrat, Rhodiumsulfat, Rhodiumacetat, Rhodium-2-ethylhexanoat, Rhodiunmalonat. Rhodiumhalogenverbindungen sind wegen der geringeren Aktivität der resultierenden Komplexe und wegen des korrosiven Verhaltens der Halogenidionen dagegen weniger geeignet. Weiterhin können Rhodiumcarbonylverbindungen wie Rh₃(CO)₁₂ oder Rh₆(CO)₁₆ oder Komplexsalze des Rhodiums, z.B. Cyclooctadienylrhodiumverbindungen eingesetzt werden. Bevorzugt ist Rhodiumoxid, besonders bevorzugt sind Rhodiumacetat und Rhodium-2-ethylhexanoat.

Es ist nicht erforderlich, die Diphosphin-Liganden der Formel I im Katalysatorsystem als einheitliche Verbindungen zu verwenden. Es können z.B. auch unterschiedliche Sulfonierungsstufen der Diphosphine und/oder Sulfonatgemische mit verschiedenen Ammonium-Kationen eingesetzt werden.

Im erfindungsgemäßen Verfahren werden olefinisch ungesättigte Verbindungen mit 2 bis 20 Kohlenstoffatomen umgesetzt, die eine oder mehrere Doppelbindungen besitzen können. Geeignet sind substituierte oder unsubstituierte Alkene mit 2 bis 20 Kohlenstoffatomen, substituierte oder unsubstituierte Diene mit 4 bis 10 Kohlenstoffatomen, substituierte oder unsubstituierte Cycloalkene oder Dicycloalkene mit 5 bis 12 Kohlenstoffatomen im Ringsystem, Ester einer ungesättigten Carbonsäure mit 3 bis 20 Kohlenstoffatomen und eines aliphatischen Alkohols mit 1 bis 18 Kohlenstoffatomen, Ester einer gesättigten Carbonsäure mit 2 bis 20 Kohlenstoffatomen und eines ungesättigten Alkohols mit 2 bis 18 Kohlenstoffatomen, ungesättigte Alkohle oder Ether mit jeweils 3 bis 20 Kohlenstoffatomen oder araliphatische Olefine mit 8 bis 20 Kohlenstoffatomen.

Bei den substituierten oder unsubstituierten Alkenen mit 2 bis 20 Kohlenstoffatomen kann es sich um geradkettige oder verzweigte Alkene mit endständiger oder innenständiger Lage der Doppelbindung handeln. Bevorzugt sind geradkettige Olefine mit 6 bis 18 Kohlenstoffatomen wie n-Hexen-1, n-Hepten-1, n-Octen-1, n-Nonen-1, n-Decen-1, n-Undecen-1, n-Dodecen-1, n-Octadecen-1 und acyclische Terpene. Geeignet sind auch verzweigte Alkene wie Diisobutylen (2,4,4-Trimethylpenten-1), Tripropylen, Tetrapropylen und Dimersol (Dibutylen).

Bevorzugte Beispiele für unsubstituierte Diene mit 4 bis 10 Kohlenstoffatomen sind 1,3-Butadien, 1,5-Hexadien und 1,9-Decadien.

Beispiele für substituierte und unsubstituierte Cycloalkene oder Dicycloalkene mit 5 bis 12 Kohlenstoffatomen im Ringsystem sind Cyclohexen, Cycloocten, Cyclooctadien, Dicyclopentadien und cyclische Terpene wie Limonen, Pinen, Camphoren und Bisabolen.

Beispielhaft für araliphatische Olefine mit 8 bis 20 Kohlenstoffatomen steht Styrol.

Als Beispiele für Ester einer ungesättigten Carbonsäure mit 3 bis 20 Kohlenstoffatomen und eines aliphatischen Alkohols mit 1 bis 18 Kohlenstoffatomen seien die Acrylsäureester und die Methacrylsäureester mit 1 - 18 Kohlenstoffatomen in der Alkohol-Komponente genannt.

Zu den Estern einer gesättigten Carbonsäure mit 2 - 20 Kohlenstoffatomen und eines ungesättigten Alkohols mit 2 - 18 Kohlenstoffatomen gehören die Vinyl- und Allylester mit 2 - 20 Kohlenstoffatomen in der Carbonsäurekomponente.

Zu den ungesättigten Alkoholen und Ethern zählen beispielsweise die Allylalkohole und Vinylether.

Bezogen auf die olefinisch ungesättigte Verbindung werden 5 bis 500 Gew.-ppm, bevorzugt 10 bis 100 Gew.-ppm und insbesondere 15 bis 50 Gew.-ppm Rhodium eingesetzt.

Die Umsetzung des Olefins mit Kohlenmonoxid und Wasserstoff erfolgt bei einer Temperatur von 80 bis 150°C, bevorzugt 100 bis 140°C und insbesondere 120 bis 130°C und unter einem Druck von 1,5 bis 30,0 MPa, vorzugsweise 2,0 bis 27,0 MPa und insbesondere 15,0 bis 25,0 MPa. Die Zusammensetzung des Synthesegases, d.h. das Volumenverhältnis von Kohlenmonoxid und Wasserstoff kann sich über weite Bereiche erstrecken und z.B. zwischen 1 : 10 bis 10 : 1 variiert werden. Im allgemeinen setzt man Gasgemische ein, in denen das Volumenverhältnis von Kohlenmonoxid und Wasserstoff etwa 1 : 1 ist oder von diesem Wert nur wenig abweicht.

Gegebenenfalls führt man das Verfahren in Anwesenheit eines organischen Lösungsmittels durch, welches unter den Bedingungen der Hydroformylierung inert ist. Geeignete Lösungsmittel sind aromatische Kohlenwasserstoffe, z.B. Toluol, ortho-Xylol, meta-Xylol, para-Xylol, Gemische isomerer Xylole, Ethylbenzol, Mesitylen, Gemische dieser Verbindungen oder aliphatische Kohlenwasserstoffe. Die Hydroformylierungsreaktion kann jedoch auch ohne Zusatz eines organischen Lösungsmittels erfolgen, wobei in diesem Fall die olefinische Ausgangsverbindung und das gebildete Hydroformylierungsprodukt als Lösungsmittel wirken.

Die Umsetzung der in flüssiger und gasförmiger Phase vorliegenden Reaktionspartner erfolgt in konventionellen Reaktoren und kann sowohl kontinuierlich als auch diskontinuierlich durchgeführt werden.

Nach Beendigung der Hydroformylierung wird das Reaktionsprodukt gekühlt und durch Entspannen von gasförmigen Bestandteilen befreit. Das dabei anfallende Reaktionsgemisch trennt man anschließend mittels Destillation, Extraktion oder Membranfiltration ab.

Die destillative Abtrennung der Hydroformylierungsprodukte vom Katalysatorsystem, z.B. unter vermindertem Druck, wird bevorzugt dann durchgeführt, wenn die Hydroformylierungsprodukte thermisch stabil sind und keine sehr hohen Siedepunkte besitzen.

Eine extraktive Entfernung des Katalysators ist sinnvoll, wenn man den Katalysator leicht in eine wasserlösliche Form überführen und dann mittels Extraktion als wäßrige Phase abtrennen kann. Mit dieser Verfahrensvariante ist eine schonende Abtrennung des Katalysators aus dem Reaktionsgemisch zu erreichen. Aus der wäßrigen Phase läßt sich der Katalysator, gegebenenfalls durch Umsalzen, wieder in eine wasserunlösliche, für den Wiedereinsatz geeignete Form umwandeln.

In einer besonders geeigneten Ausführungsform trennt man die Hydroformylierungsprodukte durch Membran-Filtration über eine semipermeablen Membran, bevorzugt über eine Polyamidmembran, insbesondere eine Polyaramidmembran, vom Katalysatorsystem ab. Hierbei leitet man das Reaktionsgemisch der Hydroformylierung, bevorzugt unter einem Druck von 1 bis 5 MPa, über die Membran und erhält ein weitestgehend vom Katalysatorsystem befreites Permeat, während sich das Katalysatorsystem aus Rhodium-Komplexverbindung sowie Diphosphin-Ligand im Retentat stark anreichert.

Die das Katalysatorsystem enthaltenden Retentate können vereinigt und, gegebenenfalls nach einer weiteren Präformierung und gegebenenfalls nach Ergänzung mit frischem Katalysator, wieder in das Hydroformylierungsverfahren eingesetzt werden. Ein mehrfacher Wiedereinsatz ist möglich, ohne daß nennenswerte Einbußen hinsichtlich Aktivität und Selektivität des Katalysatorsystems auftreten, wobei zumeist auf eine Präformierung (zusätzliche Behandlung mit Kohlenmonoxid und Wasserstoff) ebenso wie auf eine Zugabe von frischem Katalysator (Rhodium und/oder Verbindung der Formel I) verzichtet werden kann. Bei der Handhabung des Katalysatorsystems im aktiven Zustand ist generell unter Ausschluß von Luft zu arbeiten, da bereits sehr geringe Mengen Sauerstoff den Katalysator schädigen, d.h. irreversibel desaktivieren. Hierfür ist in erster Linie die Oxidation von P(III) zu P(V) in der Verbindung der Formel I verantwortlich.

Unter Verwendung der Diphosphine der Formel I werden Katalysatorsysteme zur Verfügung gestellt, die beim Einsatz in der Hydroformylierung olefinisch ungesättigter Verbindungen in homogener Phase zu ausgezeichneten Ergebnissen führen. Das erfindungsgemäße Katalyatorsystem zeichnet sich dadurch aus, daß zur Stabilisierung der darin enthaltenen Rhodium/Diphosphin-Komplexverbindungen im Vergleich zu bisher bekannten homogenen Rhodium/Phosphin-Komplexverbindungen kein hoher Überschuß an freiem, nicht komplexiertem Ligand nötig ist. Dies spiegelt sich in einem erniedrigten P : Rh-Verhältnis wider. Während im Stand der Technik zur Stabilisierung der aktiven Rhodium/Phosphin-Komplexe ein P/Rh-Verhältnis von etwa 100 : 1 eingestellt werden muß, erlaubt die Verwendung der Diphosphin-Liganden der allgemeinen Formel I eine deutliche Reduzierung der Menge an überschüssigem Ligand, bevorzugt bis auf ein P/Rh-Verhältnis von (2 - 25) : 1. Rhodium-Katalysatorsysteme mit Diphosphin-Liganden der allgemeinen Formel I sind bei der Hydroformylierung in homogener Phase unter den angegebenen Reaktionsbedingungen zudem aktiver als Katalysatorsysteme mit einem einzähnigen Liganden auf der Basis von sulfoniertem Triphenylphosphin (TPPTS) mit Alkyl- oder Arylammonium-Gegenionen. Dies ermöglicht die Verwendung einer niedrigeren Rhodium-Konzentration bei Einsatz der Rhodium/Diphosphin-Katalysatorsysteme.

Die vorgenannten Vorteile sind auch dahingehend von Bedeutung, daß sie im Rahmen der Aufarbeitung eine Abtrennung des Rhodium/Diphosphin-Katalysatorsystems vom Hydroformylierungsprodukt durch Membranfiltration besonders begünstigen. Als Folge des niedrigeren P/Rh-Verhältnisses ist die Salzkonzentration im Hydroformylierungsgemisch sehr viel niedriger als bei entsprechenden Hydroformylierungsgemischen, die mit Rhodium/Phosphin-Katalysatorsystemen des Standes der Technik erhalten werden. Bei der Durchführung der Membranfiltration werden dadurch verbesserte Flußleistungen erzielt und somit deutlich kleinere Membranflächen benötigt. Zudem ist eine gesteigerte Aufkonzentration des Retentats möglich. Wird dieses stärker aufkonzentrierte und das Katalysatorsystem enthaltende Retentat in den Hydroformylierungsreaktor zurückgeführt, so kann das Reaktorvolumen in höherem Maße für die Zufuhr der Reaktanden genutzt werden. Aufgrund des hohen Molekulargewichts der Diphosphin-Verbindungen der allgemeinen Formel I sind bei der Membranfiltration die Rückhalteraten sowohl für den freien Diphosphin-Liganden als auch für die Rhodium/Diphosphin-Komplexverbindung ausgezeichnet.

### Beispiel 1

Hydroformylierung von Propylen unter Verwendung eines Rhodium/Distearylammonium-1,3-Bis(di-m-sulfonato-phenylphosphino)propan-Katalysatorsystems.

### a) Herstellung des Distearylammoniumsalzes des 1,3-Bis(di-m-sulfonatophenylphosphino)propans.

1,3-Bis-diphenylphosphinopropan wird mit Oleum sulfoniert und das entstandene Gemisch durch Zusatz von kaltem Wasser hydrolisiert. Die P(III)Konzentration des Hydrolysegemischs beträgt 42 mmol/kg. 460,6 g des Hydrolysegemisches werden in einem Rührkolben vorgelegt. Anschließend fügt man eine Lösung von 96,6 g Distearylamin in 386 g Toluol hinzu und rührt 60 min bei 50°C. Nach Beendigung des Rührens wird die Schwefelsäure enhaltende Wasserphase abgetrennt. Durch Zusatz von 5 %iger wäßriger Natronlauge zur organischen Phase wird bei 45°C ein pH-Wert von 2,6 eingestellt. Man läßt 20 min nachreagieren. Zur besseren Phasentrennung werden 129 g Toluol zugegeben. Nach 30 min werden 662,9 g einer organischen Phase, die das Distearylammoniumsalz des 1,3-Bis(di-m-sulfonatophenylphosphino)propans enthält, abgetrennt.

### b) Hydroformylierung

Ein 5 l Rührautoklav wird mit Stickstoff gespült. In einer Glasvorlage mit Stickstoffüberlagerung werden 500 g sauerstofffreies Toluol vorgelegt. Darin werden 103,6 g der Ligandlösung aus a) sowie 0,16 mmol Rhodium in Form eines 2-Ethylhexanoatsalzes gelöst (15 ppm Rh; P/Rh-Verhältnis: 5) und unter Stickstoff in den Autoklaven überführt. Danach wird unter Rühren durch Zufuhr von Synthesegas ein Druck von 27 MPa eingestellt. Nach Erreichen einer Reaktionstemperatur von 125°C läßt man zwei Stunden präformieren. Danach werden innerhalb von 1 Stunde 1300 g Propylen aus einer Druckvorlage in den Autoklaven gepumpt. Durch Kühlung mit einem Luftgebläse wird die Temperatur von 125°C gehalten. Nach Ende der Propylen-Zufuhr läßt man noch 1 Stunde nachreagieren. Anschließend wird der Autoklav auf Raumtemperatur abgekühlt und innerhalb von 1,5 Stunden entspannt. Der Autoklaveninhalt wird dann mit Restdruck in einen 6 l Dreihalskolben mit Tauchstutzen überführt und gewogen. Aus der Gewichtszunahme errechnet sich ein Propylenumsatz von 93,9 %. Dies entspricht unter Berücksichtigung der Abgasungsverluste praktisch einem vollständigen Umsatz. Ein Teil des Produktes wird entnommen und analysiert. Es wird eine Rhodiumkonzentration von 7,96 ppm ermittelt. Die Ergebnisse der Hydroformylierung sind in Tabelle 1 dargestellt.

### c) Membranfiltration

2261,5 g des obigen Reaktionsproduktes werden über eine Labormembranfilteranlage gegeben. Als Membran wird eine Polyaramidmembran der Firma Hoechst AG (UF-PA 5(PET 100)) verwendet. Die Membran wird zunächst 15 min bei 100°C in Wasser getempert. Mittels einer Umwälzpumpe wird die Membran dann mit 150 l/h überströmt und ein Druck von 1,5 MPa eingestellt. 91,9 % des Produktes passieren die Membran als Permeat. 124,8 g verbleiben als Retentat. Die Flußleistung sinkt von anfangs 92,9 auf 59,2 l/m²h im End(Gleichgewichts)-zustand infolge Aufkonzentration.

Das Permeat wird in einer zweiten Stufe nochmals membranfiltriert. Davon passieren 93,1 % die Membran als Permeat. Die Retentatmenge beträgt 144,2 g. Die Flußleistung liegt zu Beginn bei 119, im End(Gleichgewichts)-zustand bei 75,1 l/m²h. Im Permeat wird der Gehalt an Katalysatorbestandteilen bestimmt, woraus sich, bezogen auf das eingesetzte Reaktionsprodukt, für Rhodium ein Rückhaltewert von 96,4 % ergibt.

Zum Nachweis der Recyclierfähigkeit des membranfiltrierten Katalysators werden die Retentate vereinigt und erneut, wie oben beschrieben, in die Hydroformylierung eingesetzt. Die Ergebnisse sind in der folgenden Tabelle 2 zusammengestellt.

Die hohe Flußleistung besonders im End(Gleichgewichts)zustand, die guten Rückhaltewerte sowie die geringe absolute Katalysatorkonzentration machen einen Einsatz des Katalysatorsystems im technischen Maßstab interessant.

### Vergleichsbeispiele 1a und 1b

Hydroformylierung von Propylen unter Verwendung eines Rhodium/Distearylammonium-Triphenylphosphin-trisulfonat(DSA/TPPTS)-Katalysatorsystems.

### a) Herstellung des Distearylammoniumsalzes des TPPTS

253 g einer Na-TPPTS-Lösung werden in einem Rührkolben vorgelegt und auf 65°C erwärmt. Anschließend fügt man eine Lösung von 250,3 g Distearylamin in 595 g Toluol hinzu. Innerhalb von 60 min gibt man unter Rühren 90 ml 20 %ige Schwefelsäure zu, bis ein pH-Wert von 2,6 erreicht ist, und läßt 2,5 h nachreagieren. Zur besseren Phasentrennung werden 170 g Isopropanol zugegeben. Nach 15 min werden 1037,5 g einer organischen Phase, die das Distearylammoniumsalz des TPPTS enthält, abgetrennt.

### b) Hydroformylierung

### Vergleichsbeispiel 1a:

Die Hydroformylierung erfolgt unter den gleichen Reaktionsbedingungen wie in Beispiel 1 unter Punkt b beschrieben, wobei als Ligand das DSA/TPPTS-Salz eingesetzt wird. Die Ergebnisse sind in Tabelle 1 dargestellt.

### Vergleichsbeispiel 1b:

Ein 5 l Rührautoklav wird mit Stickstoff gespült. In einer Glasvorlage mit Stickstoffüberlagerung werden 845 g der Ligandlösung aus a) sowie 1,17 mmol Rhodium in Form eines 2-Ethylhexanoatsalzes gelöst (80 ppm Rh; P/Rh-Verhältnis: 100) und unter Stickstoff in den Autoklaven überführt. Danach wird unter Rühren durch Zufuhr von Synthesegas ein Druck von 27 MPa eingestellt. Nach Erreichen einer Reaktionstemperatur von 125°C läßt man zwei Stunden präformieren. Danach werden innerhalb von 1,5 Stunden 1300 g Propylen aus einer Druckvorlage in den Autoklaven gepumpt. Durch Kühlung mit einem Luftgebläse wird die Temperatur von 125°C gehalten. Nach Ende der Propylen-Zufuhr läßt man noch 1 Stunde nachreagieren. Anschließend wird der Autoklav auf Raumtemperatur abgekühlt und innerhalb von 1,5 Stunden entspannt. Der Autoklaveninhalt wird dann mit Restdruck in einen 6 l Dreihalskolben mit Tauchstutzen überführt und gewogen. Aus der Gewichtszunahme errechnet sich ein Propylenumsatz von 85 %. Die Ergebnisse sind in Tabelle 1 dargestellt.

### c) Membranfiltration

### Vergleichsbeispiel 1a:

Wie aus Tabelle 1 hervorgeht, liefert die Hydroformylierung einen nur unzureichenden Umsatz von 54,5 %, weswegen auf eine Membranabtrennung des Katalysatorsystems verzichtet wurde.

### Vergleichsbeispiel 1b:

Das obige Reaktionsprodukt aus b) wird über eine Labormembranfilteranlage gegeben. Als Membran wird eine Polyaramidmembran der Firma Hoechst AG (UF-PA 5(PET 100)) verwendet. Die Membran wird zunächst 15 min bei 100°C in Wasser getempert. Mittels einer Umwälzpumpe wird die Membran dann mit 150 l/h überströmt und ein Druck von 1,5 MPa eingestellt. 87,3 % des Produktes passieren die Membran als Permeat. 324,2 g verbleiben als Retentat. Die Flußleistung sinkt infolge Aufkonzentration von anfangs 103 auf 10 l/m²h im Endzustand.

Das Permeat wird in einer zweiten Stufe nochmals membranfiltriert. Davon passieren 90,8 % die Membran als Permeat. Die Retentatmenge beträgt 234,9 g. Die Flußleistung liegt zu Beginn bei 136, im End(Gleichgewichts)zustand bei 51 l/m²h. Im Permeat wird der Gehalt an Katalysatorbestandteilen bestimmt, woraus sich, bezogen auf das eingesetzte Reaktionsprodukt, die in Tabelle 3 angegebenen Rückhaltewerte ergeben.

Zum Nachweis der Recyclierfähigkeit des membranfiltrierten Katalysators werden die Retentate vereinigt und erneut, wie oben beschrieben, in die Hydroformylierung eingesetzt. Die Ergebnisse sind in Tabelle 3 zusammengestellt.

Aus den Tabellen 2 und 3 geht hervor, daß für das Rhodium/DSA-TPPTS-Katalysatorsystem bei der Membranfiltration sowohl in der 1. als auch in der 2. Stufe ein deutlich stärkerer Rückgang der anfänglichen Flußleistung eintritt als bei Verwendung des Rhodium/DSA-Ghelat-Ligand-Katalysatorsystems gemäß Beispiel 1.

**Tabelle 1**

| Hydroformylierung von Propylen | | | | |
|---|---|---|---|---|
| | | Beispiel 1 | Vergleichsbeispiel 1a | Vergleichsbeispiel 1b |
| Ligand | | DSA-Chelat | DSA-TPPTS | DSA-TPPTS |
| [Rh] | ppm | 15 | 15 | 80 |
| P:Rh-Verh. | [mol/mol] | 5 | 5 | 100 |
| Umsatz | % | 93,9 | 54,5 | 85 |

Tabelle 1 zeigt, daß mit dem erfindinngsgemäßen Katalysatorsystem (Beispiel 1) bei gleichzeitig geringer Rhodium-Konzentration und niedrigem P : Rh-Verhältnis ein ausgezeichneter Umsatz erzielt wird. Unter analogen Versuchsbedingungen liefert das Katalysatorsystem mit dem DSA-TPPTS-Liganden nur einen unzureichenden Umsatz (Vergleichsbeispiel 1a). Ein höherer Umsatz ist mit diesem Katalysatorsystem nur durch eine starke Erhöhung der Rhodium-Konzentration und des P : Rh-Verhältnisses möglich (Vergleichsbeispiel 1b). Dies wirkt sich gemäß Tabelle 3 aber nachteilig auf die Flußleistung während der anschließenden Membranfiltration aus.

**Tabelle 2**

| Wiedereinsatz des membranfiltrierten Katalysators (Rhodium/DSA-Chelatligand) aus Beispiel 1 | | | | | | | |
|---|---|---|---|---|---|---|---|
| Nr. Rezirk | Rh-Menge mg abs. | Umsatz (%) | Rückhalt (%) bzg. auf Einsatz | Flußleistung (l/m²h) | | | |
| | | | Rh | 1.Stufe | | 2.Stufe | |
| | | | | Beginn | Gleichgewicht | Beginn | Gleichgewicht |
| 0 | 18.36 | 93.9 | 96,4 | 92 | 59 | 119 | 75 |
| 1* | 16,98 | 85,4 | 97,7 | 82 | 54 | 112 | 72 |

| | | | | | | | |
|---|---|---|---|---|---|---|---|
| * Es wurden 0,4 mmol Ligandlösung aus Beispiel 1 zum Permeat der 1. Stufe ergänzt | | | | | | | |

**Tabelle 3**

| Vergleichsversuch: Wiedereinsatz des membranfiltrierten Katalysators (Rh-DSA-TPPTS) | | | | | | |
|---|---|---|---|---|---|---|
| Nr. Rezirk | Umsatz (%) | Rückhalt (%) bzg. auf Einsatz | Flußleistung (l/m²h) | | | |
| | | Rh | 1.Stufe | | 2.Stufe | |
| | | | Beginn | Gleichgewicht | Beginn | Gleichgewicht |
| 0 | 85 | 96.0 | 103 | 10 | 136 | 51 |
| 1 | 90 | 99.2 | 97 | 16 | 115 | 40 |
| 2 | 83 | 99.7 | 92 | 17 | 82 | 29 |
| 3 | 92 | 98.9 | 82 | 15 | 75 | 24 |

## Patentansprüche

1. Katalysatorsysteme auf der Basis von Rhodium-Komplexverbindungen mit Diphosphin-Liganden, dadurch gekennzeichnet, daß sie als Diphosphin-Liganden Verbindungen der allgemeinen Formel I enthalten 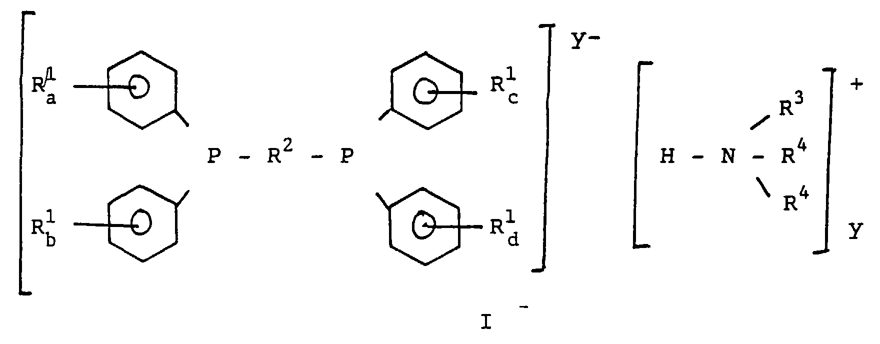 worin R¹ einen Carboxylat-(COO⁻)-, Sulfonat-(SO₃⁻), Phosphonat-(PO₃²⁻) oder 2-Aminoethanbisphosphonat-Rest ⁅NH-CH₂-CH(PO₃²⁻)₂] darstellt, R² für einen geradkettigen Alkylenrest mit 1 - 8 Kohlenstoffatomen, einen Sauerstoff enthaltenden Alkylenrest mit 2 - 6 Kohlenstoffatomen, einen Cycloalkylenrest mit 3 bis 10 Kohlenstoffatomen oder einen Rest der Formeln II, III, IV oder V 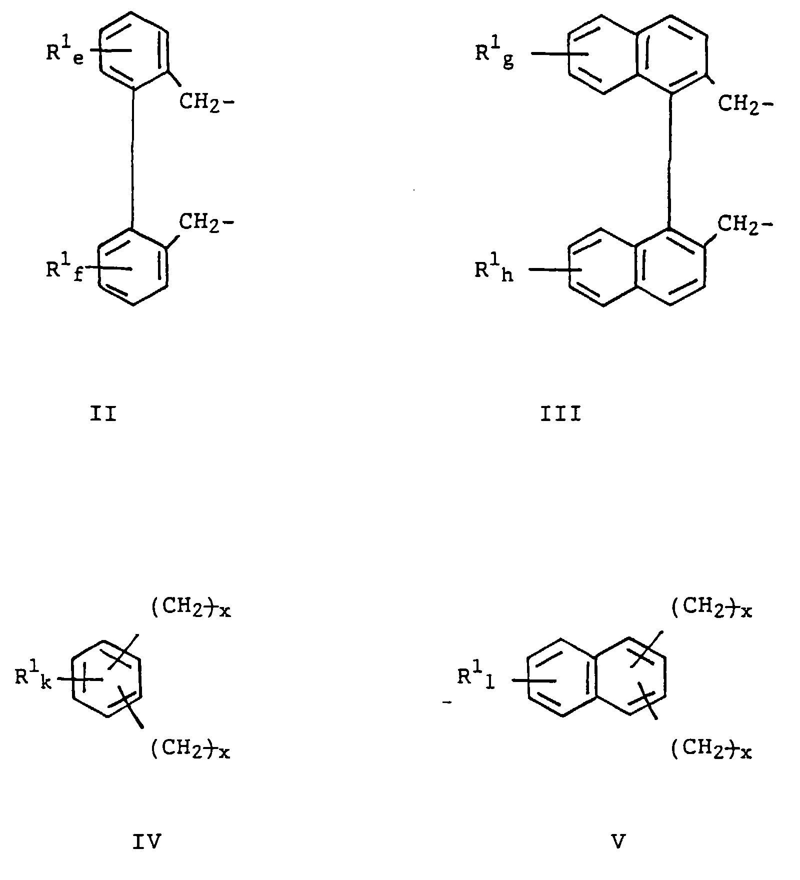 steht, a, b, c, d, e, f, g, h, k und l gleich oder verschieden und 0 oder 1 sind, wobei mindestens einer der Parameter a, b, c, d, e, f, g, h, k oder l gleich 1 sein muß, x gleich oder verschieden und 0 oder 1 ist, R³ und R⁴ gleich oder verschieden sind und für C₄ - C₂₆-Alkyl-, substituierte oder unsubstituierte C₆ - C₁₀-Aryl- oder C₆ - C₁₀-Cycloalkylreste oder einen Benzylrest stehen und R³ auch Wasserstoff bedeuten kann.

2. Katalysatorsysteme nach Anspruch 1, dadurch gekennzeichnet, daß in der allgemeinen Formel I R¹ für einen Sulfonatrest steht.

3. Katalysatorsysteme nach Anspruch 1 oder 2, dadurch gekennzeichnet, daß in der allgemeinen Formel I R² für einen geradkettigen Alkylenrest mit 1 - 5 und insbesondere 1 - 3 Kohlenstoffatomen, einen Sauerstoff enthaltenden Alkylenrest mit 2 - 4 Kohlenstoffatomen und insbesondere mit 4 Kohlenstoffatomen gemäß der Formel (̵CH₂)̵₂O(̵CH₂)̵₂, einen Cycloalkylenrest mit 6 - 10 Kohlenstoffatomen oder für einen Rest der Formel II steht.

4. Katalysatorsysteme nach einem oder mehreren der Ansprüche 1 bis 3, dadurch gekennzeichnet, daß die Summe aus a, b, c, d, e und f in den Verbindungen der Formel I, in denen R² einen Rest der Formel II darstellt, 1 - 3 beträgt.

5. Katalysatorsysteme nach einem oder mehreren der Ansprüche 1 bis 3, dadurch gekennzeichnet, daß die Summe aus a, b, c, d, g und h in den Verbindungen der Formel I, in denen R² einen Rest der Formel III darstellt, 1 - 2 beträgt.

6. Katalysatorsystem nach einem oder mehreren der Ansprüche 1 bis 3, dadurch gekennzeichnet, daß die Summe aus a, b, c, d und k bzw. a, b, c, d und l in Verbindungen der Formel I, in denen R² einen Rest der Formeln IV oder V darstellt, 1 - 3 beträgt.

7. Katalysatorsysteme nach einem oder mehreren der Ansprüche 1 bis 3, dadurch gekennzeichnet, daß die Summe aus a, b, c und d in den Verbindungen der Formel I, in denen R² die Bedeutung eines geradkettigen Alkylenrestes mit 1 - 8 Kohlenstoffatomen, eines Sauerstoff enthaltenden Alkylenrestes mit 2 - 6 Kohlenstoffatomen oder eines Cycloalkylenrestes mit 3 - 10 Kohlenstoffatomen hat, 2 - 4 beträgt.

8. Katalysatorsysteme nach einem oder mehreren der Ansprüche 1 bis 7, dadurch gekennzeichnet, daß sich die Ammonium-Kationen [H-NR³R⁴R⁴]⁺ in der allgemeinen Formel I von sekundären oder tertiären Aminen NR³R⁴R⁴ ableiten, wobei R³ und R⁴ gleich oder verschieden sind und für C₁₈ - C₂₂-Alkyl-, einen substituierten oder unsubstituierten Phenyl- oder Cyclohexylrest stehen und R³ auch Wasserstoff bedeuten kann.

9. Katalysatorsysteme nach Anspruch 8, dadurch gekennzeichnet, daß die Ammonium-Kationen [H-N-R³R⁴R⁴]⁺ in der allgemeinen Formel I insgesamt 8 - 78, bevorzugt 12 - 72, besonders bevorzugt 21 - 60 und insbesondere 36 - 54 Kohlenstoffatomen in den Resten R³ und R⁴ enthalten.

10. Katalysatorsysteme nach Anspruch 8, dadurch gekennzeichnet, daß sich die Ammonium-Kationen [H-NR³R⁴R⁴]⁺ in der allgemeinen Formel I von Di-2-ethylhexylamin, Tri-n-octylamin, Triisooctylamin, Triisononylamin, Triisodecylamin, Distearylamin, Methyldistearylamin, Tricetylamin oder Trieicosylamin ableiten.

11. Katalysatorsysteme nach einem oder mehreren der Ansprüche 1 bis 10, dadurch gekennzeichnet, daß sie je mol Rhodium 1 bis 100, bevorzugt 1 - 45, besonders bevorzugt 1 - 25 und insbesondere 1 - 10 mol des Diphosphin-Liganden der Formel I enthalten.

12. Katalysatorsysteme nach einem oder mehreren der Ansprüche 1 bis 11, dadurch gekennzeichnet, daß sie neben den Diphosphin-Liganden noch weitere zur Komplexbildung fähige Gruppen als Liganden enthalten.

13. Verfahren zur Herstellung von Aldehyden durch Umsetzung von olefinisch ungesättigten Verbindungen mit Kohlenmonoxid und Wasserstoff bei einer Temperatur von 80 bis 150°C und unter einem Druck von 1,5 - 30 MPa in Gegenwart eines Katalysatorsystems nach einem oder mehreren der Ansprüche 1 bis 12.

14. Verfahren nach Anspruch 13, dadurch gekennzeichnet, daß das Katalysatorsystem in einem dem Verfahren vorgeschalteten Schritt hergestellt wird oder in-situ während des Verfahrens gebildet wird.

15. Verfahren nach Anspruch 14, dadurch gekennzeichnet, daß zur Herstellung des Katalysatorsystems in einem vorgeschalteten Schritt die Rhodium-Komponente und das Diphosphin der Formel I, jeweils in einem organischen Lösungsmittel gelöst oder suspendiert, zusammengebracht werden und unter einem Kohlenmonoxid/Wasserstoff-Druck von 15 - 25 MPa bei einer Temperatur von 80 - 150°C mindestens 1 Stunde umgesetzt werden.

16. Verfahren nach einem oder mehreren der Ansprüche 13 bis 15, dadurch gekennzeichnet, daß als olefinisch ungesättigte Verbindungen substituierte oder unsubstituierte Alkene mit 2 bis 20 Kohlenstoffatomen, substituierte oder unsubstituierte Diene mit 4 bis 10 Kohlenstoffatomen, substituierte oder unsubstituierte Cycloalkene oder Dicycloalkene mit 5 bis 12 Kohlenstoffatomen im Ringsystem, Ester einer ungesättigten Carbonsäure mit 3 bis 20 Kohlenstoffatomen und eines aliphatischen Alkohols mit 1 bis 18 Kohlenstoffatomen, Ester einer gesättigten Carbonsäure mit 2 bis 20 Kohlenstoffatomen und eines ungesättigten Alkohols mit 2 bis 18 Kohlenstoffatomen, ungesättigte Alkohole oder Ether mit jeweils 3 bis 20 Kohlenstoffatomen oder araliphatische Olefine mit 8 bis 20 Kohlenstoffatomen verwendet werden.

17. Verfahren nach einem oder mehreren der Ansprüche 12 - 16, dadurch gekennzeichnet, daß man die Umsetzung in Gegenwart von 5 - 500, vorzugsweise 10 - 100, insbesondere 15 - 50 Gew.-ppm Rhodium, bezogen auf die olefinisch ungesättigte Verbindung, durchführt.

18. Verfahren nach einem oder mehreren der Ansprüche 13 - 17, dadurch gekennzeichnet, daß man die Umsetzung bei einer Temperatur von 100 bis 140°C, insbesondere 120 bis 130°C und unter einem Druck von 2,0 bis 27,0 MPa, insbesondere 15,0 bis 25,0 MPa durchführt.

19. Verfahren nach einem oder mehreren der Ansprüche 13 - 18, dadurch gekennzeichnet, daß das Katalysatorsystem nach der Umsetzung durch Destillation, Extraktion oder Membanfiltration vom Reaktionsgemisch abgetrennt wird.

20. Verfahren nach Anspruch 19, dadurch gekennzeichnet, daß die Membranfiltration unter Druck an einer semipermeablen Membran durchgeführt wird.

21. Verfahren nach Anspruch 20, dadurch gekennzeichnet, daß als semipermeable Membran eine Polyamidmembran, insbesondere eine Polyaramidmembran verwendet wird.
